(19) Europäisches Patentamt
European Patent Office
Office européen des brevets

(11) Veröffentlichungsnummer: **0 226 871 B1**

(12) # EUROPÄISCHE PATENTSCHRIFT

(45) Veröffentlichungstag der Patentschrift: **07.08.91**

(51) Int. Cl.⁵: **C07C 47/277**, C07C 45/60, C07F 7/18

(21) Anmeldenummer: **86116564.5**

(22) Anmeldetag: **28.11.86**

Die Akte enthält technische Angaben, die nach dem Eingang der Anmeldung eingereicht wurden und die nicht in dieser Patentschrift enthalten sind.

(54) **Ausgangsprodukte zur Herstellung von 9-beta-Chlorprostaglandinen.**

(30) Priorität: **10.12.85 DE 3543991**

(43) Veröffentlichungstag der Anmeldung:
**01.07.87 Patentblatt 87/27**

(45) Bekanntmachung des Hinweises auf die Patenterteilung:
**07.08.91 Patentblatt 91/32**

(84) Benannte Vertragsstaaten:
**AT BE CH DE ES FR GB GR IT LI LU NL SE**

(56) Entgegenhaltungen:
**EP-A- 0 053 979**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Radüchel, Bernd, Dr.**
**Gollanczstrasse 132**
**W-1000 Berlin 28(DE)**
Erfinder: **Skuballa, Werner, Dr.**
**Olwenstrasse 13**
**W-1000 Berlin 28(DE)**
Erfinder: **Vorbrüggen, Helmut, Prof.**
**Wilkestrasse 7**
**W-1000 Berlin 27(DE)**

EP 0 226 871 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft ein Verfahren zur Herstellung von 9β-Chlorprostaglandinen.

9β-Chlorprostaglandine werden in folgenden Publikationen beschrieben:

1. Chem., Biochem., Pharmacol. Act. Prostanoids, Incl.Proc. Symp. 1978 (Pub. 1979) 39-60, s.a.Chemical Abstracts 91, 107690 f
2. Prostaglandins 16, 47-65 (1978), s.a. chemical Abstracts 89, 146467 s
3. J. Am. Chem. Soc. 99, 7738 (1977)
4. Advances in Prostaglandin, Thromboxane and Leukotriene Research 14, 274 (1985)
5. Europäische Patentschrift 0 030 377

Im allgemeinen stellt man 9-Chlorprostaglandine aus den entsprechenden 9-Hydroxyverbindungen oder den entsprechenden 9-Sulfonsäureestern durch Umsetzung mit einer Chlorverbindung her. Dabei entstehen stets Gemische aus dem gewünschten 9-Chlorprostaglandin-Derivat und den $\Delta^{8,9}$ oder $\Delta^{9,10}$-Olefinen, die man bisher nur in einigen Fällen mit großem Aufwand trennen konnte.

Diese experimentellen Schwierigkeiten lassen sich umgehen, wenn man von einem kristallklaren Produkt ausgeht, das bereits Chlor enthält und das ausgehend von leicht zugänglichen Ausgangsmaterialien ohne Schwierigkeiten hergestellt werden kann.

9-Chlorprostaglandine sind wertvolle Pharmaka (s. l.c. 1 und 5). Mit Nocloprost (1) können bei Probanden Schleimhautschäden des Magens, wie sie durch nichtsteroidale entzündungshemmende Mittel verursacht werden, verhindert oder zumindest stark abgeschwächt werden.

Die Erfindung dient der vereinfachten Herstellung von wichtigen Wirkstoffen. Das erfindungsgemäße Verfahren soll anhand der Nocloprostherstellung näher erläutert werden. Für den Fachmann ist es auf der Grundlage dieser Erfindung jederzeit möglich, eine Fülle anderer 9-Chlorprostaglandine herzustellen.

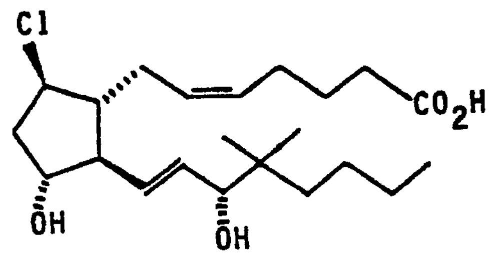

1

Die Erfindung umfaßt die Herstellung des Zwischenprodukts der Formel II für die Herstellung von 9-Chlorprostaglandinen.

Die Erfindung betrifft somit ein Verfahren zur Herstellung von (5-Chlor-3-hydroxy-cyclopentyl)-acetaldehyden der Formel II,

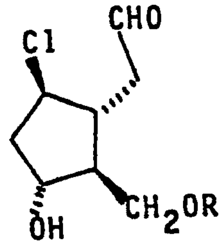

II

worin R einen Benzylrest, einen tert.-Butyl-dimethylsilylrest oder einen tert.-Butyl-diphenylsilylrest bedeuten, das dadurch gekennzeichnet ist, daß man eine Verbindung der Formel I

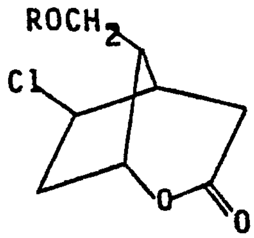

I

worin R die bereits genannten Bedeutungen hat, in Gegenwart eines Reduktionsmittels, vorzugsweise Diisobutylaluminiumhydrid, reduziert.

Die Reduktion wird in Kohlenwasserstoffen, vorzugsweise Toluol, bei Temperaturen zwischen -120° C und -20° C, vorzugsweise bei -70° C, durchgeführt.

Zur Überführung der Verbindungen der Formel II in 9β-Chlorprostaglandine wird die Hydroxygruppe in II durch Umsetzung mit Dihydropyran als Tetrahydropyranylether III geschützt. Daran schließt sich eine Abspaltung der Schutzgruppe R zu IV an. Für den Fall R = Benzyl wird die Abspaltung unter hydrogenolytischen Bedingungen mit Wasserstoff in Gegenwart von Palladiumkatalysatoren vorgenommen. Für den Fall R = tert.-Butyldimethylsilyl oder tert.-Butyl-diphenylsilyl wird die Abspaltung mit Tetrabutylammoniumfluorid durchgeführt, wobei vorzugsweise bei -20° C gearbeitet wird, um die Eliminierung von Chlorwasserstoff zu vermeiden.

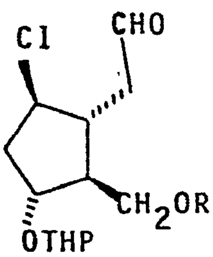

III

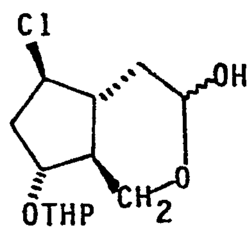

IV

Die Verbindung IV wird mit den Ylen aus 4-Carboxybutyltriphenylphosphoniumbromid zur Verbindung V umgesetzt, aus der man mit Diazomethan den Methylester VI erhält. Anschließend oxidiert man mit Collins-Reagenz oder nach der Methode von Swern zum Aldehyd VII, der durch Reaktionmit dem Anion aus 3,3-Dimethyl-2-oxoheptanphosphonsäuredimethylester in das ungesättigte Keton VIII überführt wird. An dieser Stelle kann aber auch jeder andere substituierte oder unsubstituierte 2-Oxoalkyl- oder 2-Oxoaralkylphosphonsäureester verwendet werden, so daß damit eine Fülle von 9β-Chlorprostaglandinen mit modifizierter unterer Seitenkette zugänglich wird.

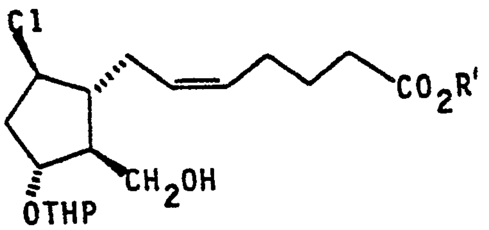

V R' = H

VI R' = $CH_3$

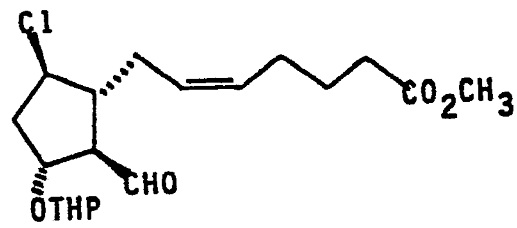

VII

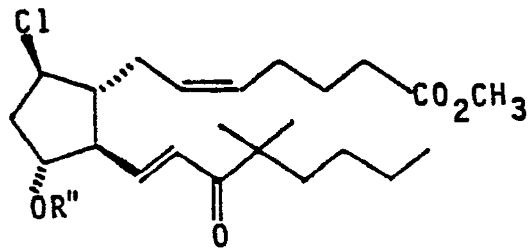

VIII R" = THP

IX R" = H

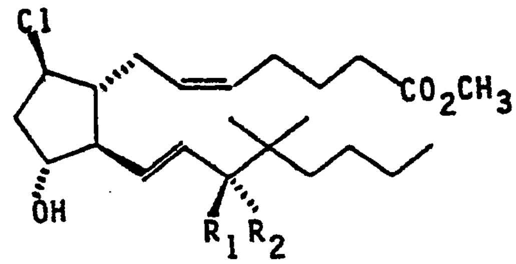

X $R_1$ = H, $R_2$ = OH

XI $R_1$ = OH, $R_2$ = H

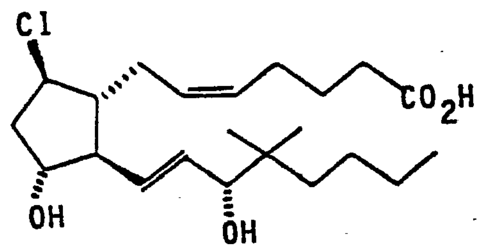

1

Durch Spaltung des Tetrahydropyranylethers in VIII mit wäßriger Essigsäure erhält man IX, das durch Umsetzen mit Reduktionsmitteln wie z.B. Natriumborhydrid, Zinkborhydrid oder Diisobutylaluminiumhydrid ein Gemisch der epimeren Alkohole X und XI ergibt. Spaltung des X-Methylesters mit wäßrigen Alkalihydroxiden, bevorzugt ist Kaliumhydroxid in einer Mischung aus Wasser und Ethanol, führt schließlich zum Nocloprost (1)

Es ist auch möglich, ausgehend von Verbindungen der Formel II zu 9β-Chlorprostaglandinen mit gesättigter oberer Seitenkette zu gelangen:

Die Umsetzung von III mit dem Ylen aus 4-Carboxybutyltriphenylphosphoniumbromid führt zu Verbindungen der Formel XII, die mit Diazomethan die entsprechenden Methylester XIII bilden. Hat R in XIII die Bedeutung eines Benzylrestes, so wird bei der Hydrierung in Gegenwart eines Palladiumkatalysator sowohl die Doppelbindung hydriert als auch der Benzyläther gespalten, und man erhält XIV. Hat R in XIII die Bedeutung eines tert. Butyl-diphenylsilyl- oder tert-Butyl-dimethylsilylrestes, so wird zunächst die Doppelbindung wie üblich hydriert, wobei man XV bzw. XVI erhält, und anschließend der Silylether mit Tetrabutylammoniumfluorid zu XIV gespalten.

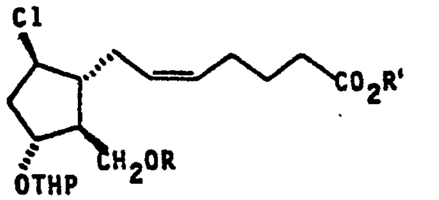

XII R' = H
XIII R' = $CH_3$

R = $CH_2C_6H_5$, tert. Butyldiphenylsilyl oder tert. Butyl-dimethylsilyl

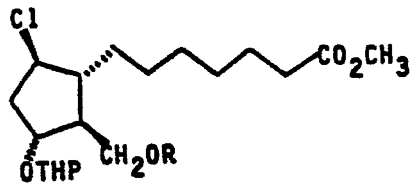

XIV R = H
XV R = tert. Butyl-diphenylsilyl
XVI R = tert. Butyl-dimethylsilyl

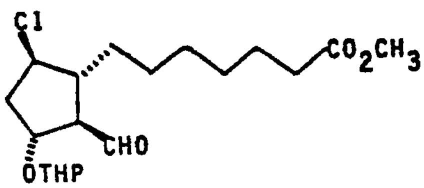

XVII

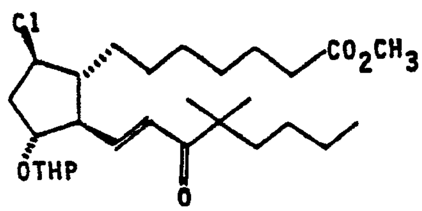

XVIII

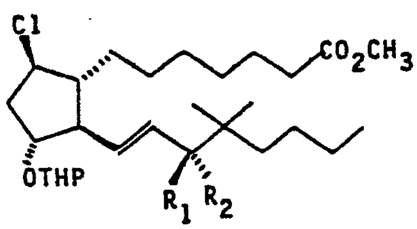

XIX $R_1$ = H, $R_2$ = OH
XX $R_1$ = OH, $R_2$ = H

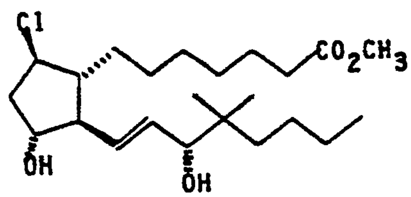

XXI

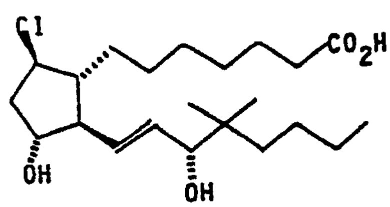

2

Man verfährt nun ähnlich wie bei der Herstellung von 1. Oxydation von XIV liefert XVII, das durch Wittig-Reaktion in XVIII überführt wird. Reduktion von XVIII gibt ein Epimerengemisch XIX und XX. Abspaltung der Schutzgruppe von XIX liefert XXI, aus dem durch Verseifen das 5,6-Dihydronocloprost 2 entsteht.

Das für die Herstellung von I benötigte Ausgangsmaterial ist auf folgendem Wege zugänglich:

5-Chlor-2-oxobicyclo[2.2.1]heptan-7-anti-carbonsäure (XXII) (s. J.Am.Chem.Soc. 95, 7522 [1973] und Tetrahedron 37 Suppl. Nr. 1, 411 [1981]) wird mit Ethylenglykol zu XXIII ketalisiert. Veresterung der Säure XXIII mit Methyljodid-Kaliumcarbonat in Aceton führt zum Ester XXIV, aus dem durch Reduktion mit Lithiumaluminiumhydrid der Alkohol XXV entsteht. Veretherung mit Benzylbromid in Gegenwart von Natriumhydrid gibt den Benzylether XXVI, aus dem durch Ketalspaltung mit wäßriger Mineralsäure das Keton XXVII entsteht. Lactonisierung von XXVII nach Baeyer-Villiger mit Peressigsäure oder m-Chlorperbenzoesäure gibt das benötigte Ausgangsmaterial I (R = Benzyl).

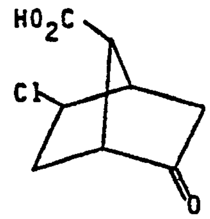

XXII

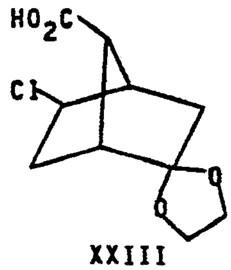

XXIII

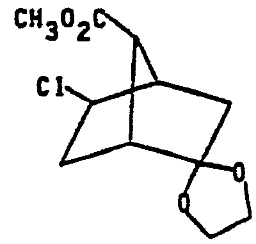

XXIV

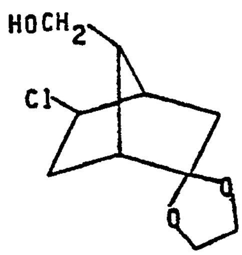

XXV

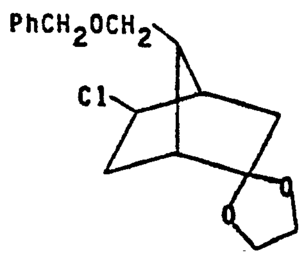

XXVI

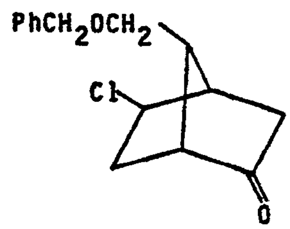

XXVII

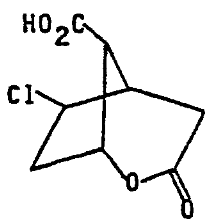

XXVIII

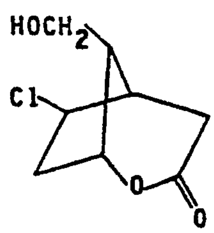

XXIX

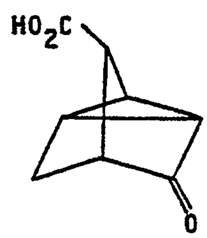

XXX

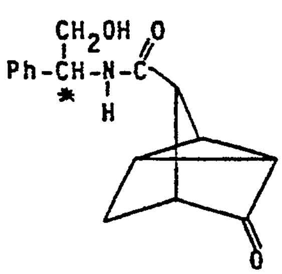

XXXI

Eine Alternative zur Herstellung von I besteht darin, daß man XXII nach Baeyer-Villiger zu XXVIII lactonisiert und XXVIII durch Umsetzung mit Chlorameisensäureester und nachfolgender Reduktion mit Natriumborhydrid in den Lactonalkohol XXIX überführt. Die Verbindung XXIX ist bekannt (Jap. Kokai 7795,671, Chem. Abst. 88, 152122 W). Nach Silylierung der Hydroxygruppe erhält man I (R = tert.-Butyl-diphenylsilyl oder tert-Butyl-dimethylsilyl).

Werden optisch aktive Endprodukte gewünscht, so kann XXII in optisch aktiver Form aus der enantiomeren reinen Säure XXX erhalten werden. Die Racematspaltung von XXX mit L-(-)-α-Methylbenzylamin ist in der Literatur beschrieben (J. Amer. Chem. Soc. 95, 7522 [1973]). Eine weitere Möglichkeit zur Racematspaltung besteht darin, daß man die racemische Säure XXX mit Chlorameisensäureester in ein

gemischtes Anhydrid überführt, das ohne Isolierung mit D-(- )-2-Amino-2-phenylethanol in das Gemisch der beiden diastereomeren Amide XXXI überführt wird. Die beiden Diastereomere können durch Säulenchromatographie getrennt werden. Durch vorsichtige Amidspaltung mit wäßriger Säure erhält man aus dem unpolareren Amid (XXXI) die für die Synthese von natürlichen Prostaglandinen einsetzbare Säure (+)-XXX. Durch Spaltung des unpolareren Amids mit kochender wäßriger Salzsäure erhält man unter gleichzeitiger Addition von Chlorwasserstoff die optisch aktive Säure (+)-XXII.

BEISPIEL 1

(±)-(1R,2S,3R,5R)-(Benzyloxymethyl-5-chlor-3-hydroxy-cyclopentyl)-acetaldehyd (II,R = $CH_2C_6H_5$)

Man löst 1 g (±)-8-anti-Benzyloxymethyl-6-exo-chlor-3-oxo-2-oxabicyclo[3.2.1]octan (I, R = $CH_2C_6H_5$) in 20 ml Toluol und versetzt bei -78° C unter Rühren tropfenweise mit 5,9 ml einer 1,2 M Lösung vom Diisobutylaluminiumhydrid in Toluol. Es wird weiter 30 Minuten bei -78° C gerührt, dann mit 0,5 ml Isopropylalkohol und 3 ml Wasser versetzt, 1 Stunde bei 20° C gerührt, filtriert und die Lösung eingedampft. Man erhält 0,97 g II(R = $CH_2C_6H_5$) als farbloses Öl.
IR: 3600, 3410 (br.), 2998, 2862, 2835, 1725, 1451, 1362, 1095/cm

BEISPIEL 2

a) (±)-(1R,2S,3R,5R)-[2-Benzyloxymethyl-5-chlor-3-(tetrahydropyran-2-yloxy)-cyclopentyl]-acetaldehyd(III) (R = $CH_2C_6H_5$)

12g II(R = $CH_2C_6H_5$) werden in 250 ml Dichlormethan gelöst und bei 0° C mit 5,2 g Dihydropyran und 40 mg p-TsOH versetzt. Man rührt 30 Minuten bei 0° C, verdünnt mit 200 ml Dichlormethan, schüttelt nacheinander mit Natriumhydrogencarbonatlösung und Sole, trocknet über Magnesiumsulfat und dampft im Vakuum ein. Man erhält 14,5 g III (R = $CH_2C_6H_5$)
IR: 2950, 2878, 2740, 1728, 1121/cm

b) (±)-(1R,2S,3R,5R)-[5-Chlor-2-hydroxymethyl-3-(tetrahydropyran-2-yloxy)-cyclopentyl]-acetaldehyd-hemiacetal (IV)

Eine Lösung von 9,5 g III (R = $CH_2C_6H_5$) in 200 ml Ethylacetat wird mit 950 mg Palladium (10%ig auf Kohle) unter Wasserstoff geschüttelt bis zur Aufnahme Von 1 Equivalent Wasserstoff. Man filtriert, dampft im Vakuum ein und kristallisiert den Rückstand aus Diethylether/Hexan (1:1) um und erhält 5,9 g IV, Fp.: 78 - 80° C
IR: 3600, 3410 (br.), 3000, 2942, 2870, 2851, 1723 (schwach) 1439, 1117, 1072, 1027, 988/cm

c) (±)-(Z)-7-[(1R,2S,3R,5R)-5-Chlor-2-hydroxymethyl-3-(tetrahydropyran -2-yloxy)-cyclopentyl]-5-heptensäure-methylester (VI)

Zu einer Lösung von 16,4 ml Hexamethyldisilazan in 50 ml Tetrahydrofuran tropft man bei 0° C 47,5 ml einer 1,6 M Lösung von Butyllithium in Hexan. Nach 15 Minuten wird diese Lösung bei 0° C zu einer gerührten Suspension von 17,3 g 4-Carboxybutyltriphenylphosphoniumbromid in 160 ml Tetrahydrofuran getropft.Man rührt noch 1 Stunde bei 0° C, kühlt dann auf -78° C ab und tropft eine Lösung von 1,80 g IV in 20 ml Tetrahydrofuran innerhalb von 20 Minuten zu. Man rührt dann 1 Stunde bei -40° C und eine weitere Stunde bei 0° C, gießt dann auf Wasser, säuert mit Citronensäure auf pH 4 an und extrahiert mit Äther. Diese Lösung der Carbonsäure V wird bei 0° C mit ätherischer Diazomethanlösung bis zur bleibenden Gelbfärbung versetzt. Man engt im Vakuum ein und reinigt den Rückstand durch Chromatographie am Kieselgel mit Hexan/10-40% Diethylether und erhält 1,45 g VI als farbloses Öl.
IR: 3430 (br.), 2998, 2948, 2870, 1729, 1437, 1130, 1028/cm

d) (±)-(5Z)-7-[(1R,2R,3R,5R)-5-Chlor-2-formyl-3-(tetrahydropyran-2-yloxy)-cyclopentyl]-5-heptensäuremethylester (VII)

Man löst 9 g Collins-Reagenz in 85 ml Dichlormethan und tropft unter Rühren innerhalb von 5 Minuten eine Lösung von 2 g VI in 25 ml Dichlormethan zu, rührt 15 Minuten, verdünnt mit 200 ml Diethylether, schüttelt mit Natriumhydrogencarbonatlösung, verdünnter Schwefelsäure und Sole trocknet über Magnesiumsulfat und dampft im Vakuum ein. Man erhält 1,72 g des Aldehyds VII als Öl.
IR: 2998, 2951, 2872, 2838, 1730, 1439, 1130, 1031/cm

e) (±)-(5Z)-7-{(1R,2R,3R,5R)-5-Chlor-2-[(E)-4,4-dimethyl-3-oxo-1-octenyl]-3-(tetrahydropyran-2-yloxy)-cyclopentyl}-5-heptensäuremethylester (VIII)

Es werden 294 mg 55%iges Natriumhydrid in 25 ml Dimethoxyethan suspendiert und 1,53 g 3,3-Dimethyl-2-oxo-heptanphosphonsäuredimethylester zugetropft. Man rührt weitere 30 Minuten, kühlt auf -20 °C ab und tropft eine Lösung von 1,98 g VII in 10 ml Dimethoxyethan und 10 ml Tetrahydrofuran zu. Nach 1 Stunde läßt man auf Raumtemperatur erwärmen, versetzt mit 0,5 ml Eisessig, verdünnt mit 200 ml Diethylether, schüttelt dreimal mit je 20 ml Sole, trocknet über Magnesiumsulfat und dampft im Vakuum zur Trockne. Der Rückstand wird an Kieselgel mit Hexan/5-25% Diethylether chromatographiert. Man erhält 1,62 g VIII als farbloses Öl.
IR: 2998, 2955, 2863, 1730, 1684, 1621, 1437, 1060, 978/cm

f) (±)-(Z)-7-{(1R,2R,3R,5R)-5-Chlor-2-[(E)-4,4-dimethyl-3-oxo-1-octenyl]-3-hydroxy-cyclopentyl}-5-heptensäuremethylester (IX)

1 g VIII werden mit 25 ml einer Mischung aus Eisessig-Wasser-Tetrahydrofuran (65/35/10) 24 Stunden bei 40 °C gerührt. Man dampft im Vakuum ein, chromatographiert an Kieselgel mit Hexan/10-50% Ethylacetat und erhält 720 mg IX als Öl.
IR: 3600, 3410 (br.), 2998, 2951, 2855, 1730, 1685, 1620, 1055, 978/cm

g) (±)-(Z)-7-{(1R,2R,3R,5R)-5-Chlor-3-hydroxy-2-[(E)-(3R)-3-hydroxy-4,4-dimethyl-1-octenyl]-cyclopentyl}-5-heptensäuremethylester (X)

Eine Lösung von 600 mg IX in 25 ml Methanol wird unter Rühren mit 60 mg Cer(III)chlorid versetzt und anschließend bei -40 °C 500 mg Natriumborhydrid zugegeben. Nach 1 Stunde zeigt die dünnschichtchromatographische Kontrolle eine vollständige Reduktion. Man versetzt tropfenweise mit 1 ml Eisessig, engt im Vakuum ein, verdünnt mit Wasser und extrahiert mehrere Male mit Dichlormethan. Die vereinigten organischen Phasen werden mit Natriumhydrogencarbonatlösung und Sole gewaschen, über Magnesiumsulfat getrocknet und im Vakuum eingedampft. Der Rückstand wird an Kieselgel mit Dichlormethan/1-10% Acetan chromatographiert. Man eluiert zunächst als unpolarere Komponente 280 mg XI und schließlich als polarere Komponente 295 mg des gewünschten Epimers X, beide als fablose Öle.
IR: (für X): 3603, 3425, (br.), 2999, 2959, 2932, 2873, 1730, 1437, 974/cm

h) (±) -(Z)-7-{(1R,2R,3R,5R)-5-chlor-3-hydroxy-2-[(E)-(3R)-3-hydroxy-4,4-dimethyl-1-octenyl]-cyclopentyl} -5-heptensäure (1, Nocloprost)

Man löst 440 mg X in 10 ml einer Mischung, die aus 5 g Kaliumhydroxid, 187 ml Ethanol und 62 ml Wasser besteht, läßt 4 Stunden bei 25 °C stehen, engt im Vakuum ein und verdünnt mit 50 ml Wasser. Die wäßrige Lösung wird mit 50 ml Ether/Hexan (1:1) extrahiert und dieser Extrakt verworfen. Die Wasserphase wird mit Citronensäure auf pH 5 angesäuert, mehrere Male mit Dichlormethan extrahiert, die vereinigten Extrakte mit Sole gewaschen, über Magnesiumsulfat getrocknet und im Vakuum eingedampft. Man erhält so 400 mg 1 als farbloses Öl.
IR: 3600, 3405 (br.), 2999, 2961, 2938, 2875, 1712, 974/cm

BEISPIEL 3

a) (±)-(Z)-7-[(1R,2S,3R,5R)-2-Benzyloxymethyl-5-chlor-3-(tetrahydropyran-2-yloxy)-cyclopentyl]-5-heptensäuremethylester (XIII, $R = CH_2C_6H_5$)

Man löst 36,7 ml Hexamethyldisilazan in 60 ml Tetrahydrofuran und tropft bei 0 °C 82 ml 1,55 M Butyllithiumlösung in Hexan zu. Man rührt noch 2 Stunden bei 0 °C und tropft diese Lösung ebenfalls bei

0 °C zu einer gerührten Suspension von 30 g 4-Carboxybutyltriphenylphosphoniumbromid in 150 ml Tetrahydrofuran. Nach 1 Stunde kühlt man auf -78 °C ab und tropft eine Lösung von 10,5 g III ($R=CH_2C_6H_5$) in 50 ml Tetrahydrofuran innerhalb von 15 Minuten zu. Man läßt dann im Verlauf von 2 Stunden auf Raumtemperatur kommen, rührt noch weitere 2 Stunden und verdünnt dann mit Wasser, stellt mit Citronensäure auf pH 4, extrahiert mit Dichlormethan, wäscht den Extrakt mit Sole, trocknet über Magnesiumsulfat, dampft im Vakuum ein und erhält die Carbonsäure XII ($R=CH_2C_6H_5$).
Zur Überführung in den Methylester löst man den Rückstand in 150 ml Diethylether und versetzt bei 0 °C mit etherischer Diazomethanlösung bis zur bleibenden Gelbfärbung, dampft im Vakuum ein und chromatographiert den Rückstand an Kieselgel mit Hexan/10-60% Ethylacetat. Man erhält 7,05 g XIII ($R=CH_2C_6H_5$) als farbloses Öl.
IR: 2998, 2943, 2855, 1731, 1452, 1438, 1075, 1028/cm

b) (±)-7-[(1R,2S,3R,5R)-5-Chlor-2-hydroxymethyl-3-(tetrahydropyran-2-yloxy)-cyclopentyl]-heptansäuremethylester (XIV)

Man schüttelt 6,50 g XIII ($R=CH_2C_6H_5$) in 150 ml Ethylacetat und 1 ml Eisessig mit 1,30 g Palladium auf Kohle (10%ig) unter einer Wasserstoffatmosphäre bis zur Aufnahme von 2 Equivalenten $H_2$, filtriert, schüttelt mit 20%iger Kaliumhydrogencarbonatlösung und Sole, trocknet über Magnesiumsulfat und dampft im Vakuum ein. Zur Reinigung chromatographiert man an Kieselgel mit Hexan/10-50% Diethylether und erhält 4,50 g XIV als farbloses Öl.
IR: 3450 (br.), 2930, 2860, 1732, 1438, 1132, 1075, 1028/cm

c) (±)-7-[(1R,2R,3R,5R)-5-Chlor-2-formyl-3-(tetrahydropyran-2-yloxy)-cyclopentyl]heptansäuremethylester (XVII)

Zu einer Suspension von 8,25 g Chrom(VI)oxid in 210 ml Dichlormethan tropft man unter intensivem Rühren 10 ml Pyridin, rührt noch 20 Minuten bei RAumtemperatur und kühlt dann wieder auf 0 °C ab. Zu diesem Reagenz tropft man rasch eine Lösung von 3,89 g XIV in 60 ml Dichlormethan. Man rührt noch 15 Minuten, verdünnt dann mit eiskaltem Diethylether und schüttelt nacheinander mit gesättigter Kaliumhydrogencarbonatlösung, 10%iger Schwefelsäure und Sole, trocknet über Magnesiumsulfat und dampft im Vakuum ein. Man erhält 3,68 g XVII als braunes Öl.
IR: 2935, 2860, 2720, 1725, 1438, 1125, 1075, 1028/cm

d) (±)-7-[(1R,2R,3R,5R)-5-Chlor-2-[(E)-4,4-dimethyl-3-oxo-1-octenyl]-3(tetrahydropyran-2-yloxy)-cyclopentyl]-heptansäuremethylester (XVIII)

Zu einer Suspension von 542 mg Natriumhydrid in 40 ml Dimethoxyethan tropft man bei 0 °C 2,82 g 3,3-Dimethyl-2-oxo-heptanphosphonsäuredimethylester gelöst in 40 ml Dimethoxyethan. Man rührt 30 Minuten bei Raumtemperatur, kühlt dann auf -15 °C ab und tropft 3,65 g XVII gelöst in 45 ml Dimethoxyethan innerhalb von 10 Minuten zu. Anschließend wurde 1 Stunde bei 0 °C und 1 Stunde bei Raumtemperatur gerührt. Zur Aufarbeitung wurde bei 0 °C mit 1 ml Eisessig und 500 ml Wasser versetzt, dann mehrmals mit Diethylether extrahiert und die vereinigten Extrakte nacheinander mit Natriumhydrogencarbonatlösung und Sole geschüttelt, über Magnesiumsulfat getrocknet und im Vakuum eingedampft. Der Rückstand wurde an Kieselgel mit Hexan/10-40% Diethylether chromatographiert. Man erhielt 3,68 g XVIII als farbloses Öl.
IR: 2932, 2859, 1731, 1686, 1621, 1465, 1438, 1075, 1028, 980/cm

e) (±)-7-[(1R,2R,3R,5R)-5-Chlor-2-[(E)-(3R)-3-hydroxy-4,4-dimethyl-1-octenyl]-3-(tetrahydropyran-2-yloxy)-cyclopentyl]-heptansäuremethylester (XIX)

Zu einer Lösung von 1 g XVIII in 50 ml Methanol gibt man bei -20 °C 1 g Natriumborhydrid und nach 30 Minuten noch einmal 1 g Natriumhydrid. Nach weiteren 30 Minuten gießt man die Reaktionslösung langsam auf einen pH 4 Citratpuffer, schüttelt dann mehrmals mit Dichlormethan aus, trocknet über Magnesiumsulfat und dampft im Vakuum ein. Der Rückstand besteht aus einem Gemisch der beiden epimeren Alkohole XIX und XX.
Zur Trennung chromatographiert man an Kieselgel mit Hexan/10-50% Diethylether und erhält als unpolarere Komponente 450 mg XX und als polarere Komponente 510 mg XIX, beide als farblose Öle.
IR: (für XIX): 3600, 3460 (br.), 2930, 2861, 1730, 1438, 1075, 1028, 975/cm

f) (±)-7-{(1R,2R,3R,5R)-5-Chlor-3-hydroxy-2-[(E)-(3R)-3-hydroxy-4,4-dimethyl-1-octenyl]-cyclopentyl}-heptansäuremethylester (XXI)

Man rührt 1,02 g XIX 48 Stunden mit 10 ml einer Mischung aus Eisessig-Wasser-Tetrahydrofuran (65/35/10), engt im Vakuum ein, verdünnt mit 150 ml Dichlormethan, schüttelt nacheinander mit Kaliumhydrogencarbonatlösung und Sole, trocknet über Magnesiumsulfat und dampft im Vakuum ein. Den Rückstand chromatographiert man an Kieselgel mit Hexan/10-50% Diethylether und erhält 710 mg XXI als farbloses Öl.
IR: 3600, 3410 (br.), 2952, 2860, 1731, 1438, 974/cm

g) (±)-7-{(1R,2R,3R,5R)-5-Chlor-3-hydroxy-2-[(E)-(3R)-3-hydroxy-4,4-dimethyl-1-octenyl]-cyclopentyl}-heptansäure (2, 5,6-Dihydronocloprost)

Zu einer Lösung von 600 mg XXI in 8 ml Ethanol gibt man 8 ml 0,7 N Kalilauge. Nach 3 Stunden engt man im Vakuum ein, verdünnt mit 80 ml Wasser und extrahiert mit 50 ml Diethylether. Dieser Extrakt wird verworfen. Die Wasserphase wird mit Citronensäure auf pH 4 eingestellt und mehrmals mit Dichlormethan extrahiert. Die vereinigten Extrakte werden getrocknet und im Vakuum eingedampft. Man erhält 540 mg 2 als farbloses Öl.
IR: 3600, 3420 (br.), 2953, 2860, 1712, 1465, 1075, 974/cm

BEISPIEL 4

a) (±)-5-exo-Chlor-2,2-ethylendioxy-bicyclo-[2,2,1]heptan-7-anti-carbonsäure (XXIII)

36,5 g (±)-5-exo-Chlor-2-oxobicyclo[2,2,1]heptan-7-anti-carbonsäure (XXIII) werden in 500 ml Benzol mit 13 ml Ethylenglykol und 365 mg p-Toluolsulfonsäure 3 Stunden unter Rückfluß erhitzt, wobei das entstehende Wasser durch einen Abscheider abgetrennt wird. Man kühlt ab auf 5 °C, saugt die ausgefallenen Kristalle ab, wäscht mit Toluol/Hexan (2:1) nach und trocknet den Kristallbrei im Vakuum. Man erhält 39,4 g XXIII, Fp.: 168-169 °C.
IR: 3420, 2995, 2895, 1715, 1330, 1158, 1112, 1078, 1022, 958, 905/cm

b) (±)-5-exo-Chlor-2,2-ethylendioxy-bicyclo[2,2,1]heptan-7-anti-carbonsäuremethylester (XXIV)

6,98 g XXIII werden in 50 ml Dimethylformamid gelöst, mit 3,1 ml Methyljodid und 4,14 g Kaliumcarbonat versetzt und 24 Stunden bei Raumtemperatur gerührt. Man verdünnt dann mit 500 ml Eiswasser und schüttelt zweimal mit je 200 ml Pentan/Diethylether (1:1) aus, wäscht die organische Phase mit 50 ml Sole, trocknet über Magnesiumsulfat und dampft im Vakuum ein. Der Rückstand wird aus Hexan/Diethylether umkristallisiert. Man erhält 5,35 g XXIV, Fp.: 71-72 °C
IR: 2962, 2902, 1737, 1440, 1332, 1159, 1110, 1078, 1038, 1023, 960, 915, 903/cm

c) (±)-5-exo-Chlor-2,2-ethylendioxy-7-anti-hydroxymethyl-bicyclo[2,2,1]heptan (XXV)

Zu einer Suspension von 9,11 g Lithiumalanat in 1 l Diethylether tropft man innerhalb von 30 Minuten unter Rühren 39,5 g XXIV gelöst in 1 l Äther. Anschließend wird 2 Stunden unter Rückfluss erhitzt, auf 0 °C abgekühlt und langsam 100 ml Ethylacetat zugetropft, 1 Stunde gerührt, mit 0,5 l Diethylether verdünnt und vorsichtig 20 ml Wasser zugetropft. Nach 30 Minuten wird filtriert, über Magnesiumsulfat getrocknet und im Vakuum eingedampft. Man erhält 33,0 g XXV als farbloses Öl.
IR:3600, 3470 (br.), 2980, 1330, 1115, 1072, 1023, 980, 951, 907, 845/cm

d) (±)-7-anti-Benzyloxymethyl-5-exo-chlor-2,2-ethylendioxy-bicyclo[2,2,1]heptan (XXVI)

12 g Natriumhydrid (55%ig) werden mit 100 ml Hexan gerührt, dann Hexan abdekantiert und der Rückstand mit 400 ml Dimethylformamid versetzt. Zu dieser Suspension tropft man unter Rühren 48,7 g

XXV gelöst in 40 ml Dimethylformamid. Man rührt 1 Stunde und tropft dann 41,9 g Benzylbromid zu und rührt 24 Stunden. Man versetzt vorsichtig mit 1,5 l Wasser und extrahiert mehrmals mit Pentan. Die vereinigten Extrakte werden mit Sole gewaschen, über Magnesiumsulfat getrocknet und im Vakuum eingeengt. Der Rückstand wird bei 0,1 Torr im Kugelrohr bei einer Badtemperatur von 100 °C destilliert. Man erhält 45,7 g XXVI als farblose Flüssigkeit.
IR: 2976, 2878, 1451, 1327, 1098/cm

e) (±)-7-anti-Benzyloxymethyl-5-exo-chlor-2-oxo-bicyclo [2,2,1]heptan (XXVII)

Eine Lösung von 55 g XXVI in 1,10 l Aceton wird mit 220 ml 10vol %ige Schwefelsäure versetzt und 3 Stunden unter Rückfluß erhitzt. Man engt im Vakuum ein, verdünnt mit 500 ml Wasser, extrahiert mehrmals mit Dichlormethan, wäscht den Extrakt mit Sole, trocknet über Magnesiumsulfat und engt im Vakuum ein. Der flüssige Rückstand wird bei 0,1 Torr und 90 °C Badetemperatur im Kugelrohr destilliert. Man erhält 43 g XXVII als farblose Flüssigkeit.
IR: 3005, 2920, 2861, 1749, 1452, 1098/cm

f) (±)-8-anti-Benzyloxymethyl-6-exo-chlor-3-oxo-2-oxabicyclo [3,2,1]octan (I,R = $CH_2C_6H_5$)

Eine Lösung von 20 g XXVII in 400 ml Dichlormethan wird bei 0 °C 3 Stunden mit 10,8 g Natriumhydrogencarbonat und 29,3 g m-Chlorperbenzoesäure (85%ig) gerührt. Man versetzt dann mit 400 ml 5%iger Natriumhydrogensulfitlösung, schüttelt durch, extrahiert die Wasserphase mit Dichlormethan, vereinigt die organischen Phasen, wäscht mit Natriumhydrogencarbonatlösung und Sole, trocknet und dampft im Vakuum ein. Der Rückstand wird an Kieselgel mit Hexan/Ethylacetat (7:3) chromatographiert und die sauberen Fraktionen aus Diethylether/Hexan (1:1) umkristallisiert. Man erhält 11,9 g I (R = $CH_2C_6H_5$), Fp.: 74-75 °C
IR: 3030, 2998, 2861, 1738, 1452, 1363, 1164, 1119, 1032/cm

BEISPIEL 5

(+)-5-exo-Chlor-2-oxobicyclo[2,2,1]heptan-7-anti-carbonsäure (+)- (XXII)

a) Eine Lösung von 15 g 2-Oxotricyclo [2,2,1,$0^{3,5}$]heptan-7-anticarbonsäure in 600 ml Aceton wird bei 0 °C mit 11,47 g Triäthylamin und 15,49 g Chlorameisensäureisobutylester versetzt. Nach 30 Minuten tropft man 16,91 g D-(-)-2-Amino-2-phenylethanol gelöst in einer Mischung aus 138 ml Aceton und 138 ml Acetonitril. Nach 2 Stunden Rühren bei Raumtemperatur wird im Vakuum eingeengt, mit 1,5 l Dichlormethan verdünnt und dreimal mit je 100 ml Sole gewaschen, über Magnesiumsulfat getrocknet und im Vakuum eingedampft. Der Rückstand wird an Kieselgel mit Hexan/Ethylacetat (20-80%) chromatographiert. Man erhält 6,10 g des unpolareren Amids 2-Oxotricyclo[2,2.1.$0^{3,5}$]heptan-7-anti-[D-(-)-2-amino-2-phenyläthanol]-carboxamid (XXXI) und 5,95 g des polareren Amids (XXXI (beides zähe Öle) neben 4 g Gemisch der beiden Amide. Im Dünnschichtchromatogramm zeigen die getrennten Amide einheitliche Flecken an Kieselgelplatten (Laufmittel: Ethylacetat/Hexan 9:1) mit Rf-Werten von 0,31 resp. 0,24.
Die IR-Spektren beider Amide sind nahezu identisch, während im NMR-Spektrum Unterschiede sichtbar sind.
IR (pol.Amid): 3600, 3435, 3030, 2958, 2890, 1760, 1669, 1508, 950, 841, 702/cm
b) 3,68 g des unpolareren Amids XXXI werden mit 40 ml konzentrierter Salzsäure 5 Stunden unter Rückfluss erhitzt. Man verdünnt dann mit 100 ml Wasser, extrahiert dreimal mit je 50 ml Ethylacetat, wäscht die vereinigten Extrakte mit 20 ml Sole, trocknet über Magnesiumsulfat und dampft im Vakuum ein. Den Rückstand kristallisiert man aus Ethylacetat/Hexan um und erhält 2,10 g (+)-XXII, Fp.:151-152 °C, $[\alpha]_D$ + 13,6 ° (C = 1 in Methanol).
IR: 3510, 3005, 2960, 1761, 1712, 1292, 949, 840/cm

BEISPIEL 6

Führt man den gleichen Reaktionsschritt mit dem polareren Amid XXXI durch, so erhält man (-)-XXII, Fp.:156-157 °C, $[\alpha]_D$-13,0 ° (C = 1 in Methanol)

BEISPIEL 7

Geht man von der nach Beispiel 6 hergestellten Carbonsäure (+)-XXII aus und setzt sie entsprechend Beispiel 2 (für die Synthese von 1) um, so erhält man Nocloprost in der optisch aktiven physiologisch wirksamen linksdrehenden Form, $[\alpha]_D$ -10,2° (C=0,5 in Dioxan).

## Patentansprüche

1. Verfahren zur Herstellung von (5-Chlor-3-hydroxy-cyclopentyl)-acetaldehyden der Formel II

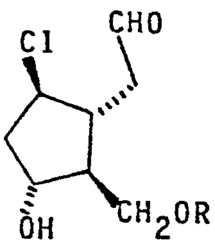

II

worin R einen Benzylrest, einen tert.-Butyl-dimethyl-silylrest oder einen tert.-Butyl-diphenyl-silylrest bedeutet,
das dadurch gekennzeichnet ist, daß man eine Verbindung der Formel I

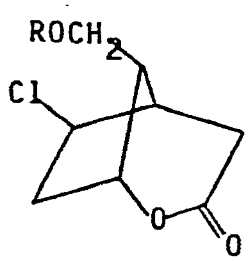

I

worin R die bereits genannten Bedeutungen hat, in Gegenwart eines Reduktionsmittels reduziert.

2. Verfahren nach Anspruch 1, dadurch gekennzeichnet, daß man als Reduktionsmittel Diisobutylaluminiumhydrid verwendet.

## Claims

1. Process for the preparation of (5-chloro-3-hydroxy-cyclopentyl)-acetaldehydes of formula II

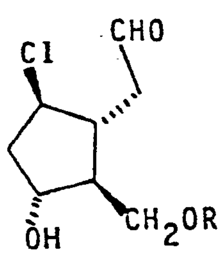

II

in which R is a benzyl radical, a tert.-butyldimethylsilyl radical or a tert.-butyldiphenylsilyl radical, which process is characterised in that a compound of formula I

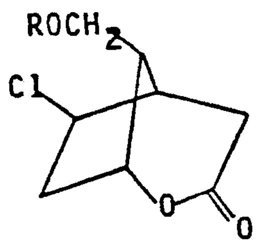

I,

in which R has the meanings already mentioned, is reduced in the presence of a reducing agent.

2. Process according to claim 1, characterised in that diisobutylaluminium hydride is used as reducing agent.

## Revendications

1. Procédé pour préparer des (chloro-5 hydroxy-3 cyclopentyl)-acétaldéhydes répondant à la formule II :

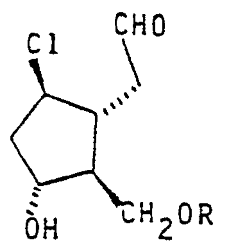

II

dans laquelle R représente un radical benzyle, un radical tert-butyl-diméthyl-silyle ou un radical tert-butyl-diphényl-silyle,
procédé caractérisé en ce qu'on réduit un composé répondant à la formule I :

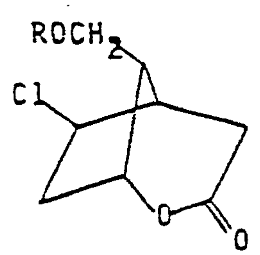

I

dans laquelle R a la signification indiquée ci-dessus, en présence d'un réducteur.

2. Procédé selon la revendication 1 caractérisé en ce qu'on utilise l'hydrure de di-isobutyl-aluminium comme réducteur.